# EUROPEAN PATENT APPLICATION

(11) **EP 3 725 889 A1**
(43) Date of publication of application: **21.10.2020**
(21) Application number: 18887417.6
(22) Date of filing: 10.12.2018
(51) Int. Cl.: C12P 7/10, C12P 19/02, C13K 1/02

(54) **METHOD FOR PRODUCING SACCHARIFIED SOLUTION BY ENZYMATIC PROCESS USING CELLULOSE-BASED BIOMASS AS RAW MATERIAL**

(30) Priority: 11.12.2017 JP 2017236922
(71) Applicant: Kawasaki Jukogyo Kabushiki Kaisha, Hyogo 650-8670 (JP)
(72) Inventor: NISHINO, Takashi, Hyogo 650-8670 (JP); IZUMI, Noriaki, Hyogo 650-8670 (JP); TAJIRI, Hironori, Hyogo 650-8670 (JP); TSUJITA, Shoji, Hyogo 650-8670 (JP); ODA, Asuka, Hyogo 650-8670 (JP); MASAMOTO, Manabu, Hyogo 650-8670 (JP); WARATANI, Yusuke, Hyogo 650-8670 (JP)
(74) Representative: Leinweber & Zimmermann
(86) International application number: PCT/JP2018/045298
(87) International publication number: WO 2019/117080

(57) **Abstract**

An object of the present invention is to provide a saccharified solution production method in which cellulosic biomass is solubilized by enzymatic hydrolysis of cellulose contained in the cellulosic biomass, the method being adapted to quickly solubilize the biomass and give a slurry while keeping a high solids concentration in a reaction vessel. The biomass pulverized and an aqueous solution containing a cellulose-hydrolyzing enzyme are mixed in one reaction vessel including no baffle plate disposed in its interior, and the cellulosic biomass is solubilized under stirring. Afterwards, the contents of the reaction vessel are transferred to another reaction vessel including a baffle plate disposed in its interior, and enzymatic hydrolysis of cellulose is allowed to proceed. The solids concentration in the first reaction vessel is preferably 15 to 30% by mass. The efficiency of stirring during enzymatic hydrolysis of cellulose is enhanced, and thus the amount of sugar production can be increased.

## Description

### Technical Field

The present invention relates to a method used to produce a biochemical substance such as ethanol (bioethanol) or polylactic acid from sugars by a fermentation technique such as alcoholic fermentation or lactic acid fermentation and particularly to a method for producing a saccharified solution by hydrolyzing cellulosic biomass with a hydrolytic enzyme.

### Background Art

In the context of biomass energy applications, there is an attempt to produce a saccharified solution by hydrolyzing cellulose or hemicellulose, which is a major constituent of plants, and obtain ethanol by subjecting the sugars of the saccharified solution to alcoholic fermentation. The planned goal of this attempt is the use of the obtained ethanol as fuel, such as, typically, the use as part of an automotive fuel mixture or as an alternative to gasoline.

Nowadays, a technique is also industrially used which consists of: producing L-lactic acid by subjecting to lactic acid fermentation a saccharified solution resulting from hydrolysis of cellulose or hemicellulose; and polymerizing the L-lactic acid to produce polylactic acid which is a kind of bio-based polymer. Polylactic acid is attracting attention as a biodegradable plastic.

Major constituents of plants include cellulose (a polymer of glucose which is a C6 monosaccharide containing six carbon atoms), hemicellulose (a polymer of a C5 monosaccharide containing five carbon atoms and a C6 monosaccharide), lignin, and starch. Ethanol is produced by using as a raw material a sugar such as a C5 monosaccharide, a C6 monosaccharide, or an oligosaccharide which is a combination of C5 and C6 monosaccharides and exposing the raw material to the fermentation activity of a microorganism such as a yeast fungus.

The following three methods are being considered for industrial use in hydrolysis of cellulosic biomass such as cellulose or hemicellulose into sugars: 1) a method in which cellulosic biomass is hydrolyzed by oxidizing power of a strong acid such as sulfuric acid; 2) a method in which cellulosic biomass is hydrolyzed with an enzyme; 3) a method utilizing the oxidizing power of supercritical water or subcritical water. Among these methods, the enzymatic hydrolysis method 2), although requiring a longer hydrolysis time than the other hydrolysis methods, has the advantages of involving a lower production equipment cost and a lower running cost, allowing the process to proceed at normal temperature and pressure, and having a lower likelihood of excessive sugar hydrolysis.

Patent Literature 1 states that, when lignocellulosic biomass subjected to lignin removal or swelling treatment is transported to a reaction chamber for enzymatic saccharification reaction by means such as a transportation container, the lignocellulosic biomass can be contaminated with bacteria during transfer to the transportation container, and that sugars produced by an enzymatic saccharification reaction of the contaminated lignocellulosic biomass will be consumed by the bacteria.

To solve this problem, Patent Literature 1 discloses a method for processing lignocellulosic biomass by pre-treating lignocellulosic biomass in a reaction chamber and then transferring the biomass to another reaction chamber for enzymatic saccharification to yield a sugar solution. The method includes: a pre-treatment step of pre-treating the lignocellulosic biomass in a first reaction chamber to dissociate lignin from the lignocellulosic biomass or swell the lignocellulosic biomass, thereby yielding a first processed product; a first saccharification treatment step of subjecting the first processed product yielded in the pre-treatment step to a partial enzymatic saccharification reaction in a second reaction chamber to yield a second flowable processed product; a transfer step of transferring the second processed product yielded in the first saccharification treatment step to a third reaction chamber without contacting the second processed product with the outside air; and a second saccharification treatment step of subjecting the second processed product transferred in the transfer step to an enzymatic saccharification reaction in the third reaction chamber to yield a sugar solution.

Patent Literature 2 discloses a lignocellulosic biomass hydrolysis process which does not need shearing and can hydrolyze cellulose in a very short time. The process includes the steps of: A) contacting a lignocellulosic feedstock, the feedstock comprised of biomass having a dry content and water, with at least a portion of a solvent, the solvent comprised of water soluble hydrolyzed species, wherein at least some of the water soluble hydrolyzed species are the same as the water soluble hydrolyzed species obtainable from the hydrolysis of the biomass in the feedstock; and B) maintaining the contact between the feedstock of the feedstock stream and the solvent at a temperature in the range of 20 to 200°C for a time in the range of 5 minutes to 72 hours to create a hydrolyzed product from the biomass in the feedstock.

Patent Literature 3 discloses a method for producing sugars including glucose as a major constituent by an enzymatic saccharification reaction, the method being adapted to increase the production of the sugars even if the amount of the enzyme used is small. Specifically, the method is a method for producing sugars including glucose as a major constituent by hydrolyzing cellulose and/or hemicellulose with a cellulose-hydrolyzing enzyme, the method including: mixing cellulose and/or hemicellulose with an aqueous enzyme solution containing a cellulose-hydrolyzing enzyme to prepare a mixture of the cellulose and/or hemicellulose and the aqueous enzyme solution; and stirring the mixture in a reaction chamber to carry out an enzymatic saccharification reaction in which the cellulose and/or hemicellulose is saccharified with the cellulose-hydrolyzing enzyme, wherein a stirring power Y (W/m³) applied to the mixture and a percentage X (w/v%) of the cellulose and/or hemicellulose added to the aqueous enzyme solution satisfy the following relationship: Y ≤ -0.0125X² + 1.195X + 23.25.

When cellulose contained in cellulosic biomass is hydrolyzed with a hydrolytic enzyme such as cellulase, it is ideal to prepare an aqueous solution containing cellulose at as high a concentration as possible and subject the aqueous solution to hydrolysis to produce a highly concentrated saccharified solution. However, when the solids concentration (biomass concentration) in the reaction vessel is high at the beginning of hydrolysis of cellulose, stirring of the contents of the reaction vessel is difficult due to the high viscosity of the contents. The use of a stirring device capable of applying high stirring power entails increases in equipment cost and operation cost.

Patent Literature 4 discloses a technique for solving such a problem, and the technique is a saccharified solution production method in which cellulosic biomass is solubilized by enzymatic hydrolysis of cellulose contained in the cellulosic biomass, the method being adapted to quickly achieve solubilization of the cellulosic biomass and give a slurry while keeping a high solids concentration in a reaction vessel at the beginning of the enzymatic hydrolysis. In this production method, at the beginning of mixing of pulverized cellulosic biomass and an aqueous solution containing a cellulose-hydrolyzing enzyme in a reaction vessel, the aqueous solution is first poured into the reaction vessel, and subsequently the pulverized cellulosic biomass is introduced stepwise while the contents of the reaction vessel are stirred.

### Citation List

### Patent Literature

PTL 1: WO 2011/142290
PTL 2: Japanese National Phase PCT Laid-Open Publication No. 2012-521778
PTL 3: Japanese Laid-Open Patent Application Publication No. 2012-139144
PTL 4: Japanese Laid-Open Patent Application Publication No. 2017-139973

### Summary of Invention

### Technical Problem

As stated above, when cellulose contained in cellulosic biomass is hydrolyzed with a hydrolytic enzyme such as cellulase, it is ideal to prepare an aqueous solution containing cellulose at as high a concentration as possible and subject the aqueous solution to hydrolysis to produce a highly concentrated saccharified solution, but in this case stirring of the contents of the reaction vessel is difficult due to the high viscosity of the contents. Insufficient stirring of the contents of the reaction vessel leads to an increase in the time required to hydrolyze part of cellulose contained in the cellulosic biomass and solubilize the cellulosic biomass.

Patent Literatures 1 to 3 are silent on how to address solubilization of cellulosic biomass at the beginning of enzymatic hydrolysis of cellulose. Patent Literature 4 fails to mention the type of the reaction vessel.

An object of the present invention is to provide a saccharified solution production method in which cellulosic biomass is solubilized by enzymatic hydrolysis of cellulose contained in the cellulosic biomass, the method being adapted to improve the efficiency of the enzymatic hydrolysis of the cellulosic biomass while keeping a high solids concentration in a reaction vessel, and thereby increase the amount of sugar production.

### Solution to Problem

To solve the problems as mentioned above, the present inventors have conducted intensive studies. In these studies, the inventors tried a method in which a reaction vessel including no baffle plate disposed in its interior is used when pulverized cellulosic biomass and an aqueous solution containing a cellulose-hydrolyzing enzyme are mixed for solubilization of the cellulosic biomass and in which, after the viscosity of the contents of the reaction vessel is reduced with the progress of solubilization, the contents are transferred to another reaction vessel including a baffle plate disposed in its interior. As a result, the inventors have found that, after the solubilization which reduces the viscosity of the reaction vessel contents, stirring of the reaction vessel contents can be promoted by the presence of the baffle plate in the reaction vessel to improve the efficiency of cellulose hydrolysis without having to use a specialized stirring device. Thus, the inventors have completed the present invention.

In particular, the present invention relates to a saccharified solution production method for obtaining a saccharified solution by enzymatic hydrolysis of cellulosic biomass, the method including the steps of:
A) hydrolyzing hemicellulose contained in the cellulosic biomass by hot water treatment or with a hemicellulose-hydrolyzing enzyme;
B) mixing a solid residue, from which hemicellulose has been removed, with an aqueous solution containing a cellulose-hydrolyzing enzyme in a first reaction vessel, adjusting the resulting mixture to a solids concentration of 15 to 30% by mass, and then stirring the mixture with a first stirring device disposed in an interior of the first reaction vessel to solubilize the solid residue from which hemicellulose has been removed;
C) transferring the contents of the first reaction vessel to a second reaction vessel after the step B and stirring the contents of the second reaction vessel with a second stirring device disposed in an interior of the second reaction vessel to allow a hydrolysis reaction to proceed; and
D) withdrawing a slurry obtained as a result of the hydrolysis reaction from the second reaction vessel, wherein
the first reaction vessel includes no baffle plate disposed in the interior thereof, and
the second reaction vessel includes a baffle plate disposed in the interior thereof.

Preferably, the step B is continued for a time of 5 minutes to 24 hours while the contents of the first reaction vessel are held at a temperature of 20 to 90°C. Preferably, the step C is continued for a time of 5 minutes to 120 hours while the contents of the second reaction vessel are held at a temperature of 20 to 90°C.

Preferably, the first stirring device includes a plurality of impellers disposed at least in the interior of the first reaction vessel, and the second stirring device includes a single impeller disposed at least in the interior of the second reaction vessel.

Preferably, each of the first stirring device and the second stirring device includes an impeller selected from the group consisting of a helical ribbon impeller, a double helical ribbon impeller, a Maxblend impeller, an inclined paddle impeller, and an anchor impeller, and the impeller of the first stirring device is rotated at an impeller tip speed of 0.5 to 5.0 m/s.

The saccharified solution production method of the present invention may further include a step A1 of removing lignin contained in the cellulosic biomass before the step B.

### Advantageous Effects of Invention

With the saccharified solution production method of the present invention, hydrolysis process of cellulose contained in pulverized biomass can be efficiently accomplished.

### Brief Description of Drawings

FIG. 1 shows a basic flow of a process of producing a saccharified solution from cellulosic biomass by an enzymatic technique.
FIGS. 2A and 2B are schematic configuration diagrams showing examples of a first reaction vessel.
FIG. 3A and 3B are schematic configuration diagrams showing examples of a second reaction vessel.
FIG. 4 is a graph plotted for a relationship between the stirring time and the sugar concentration of a saccharified solution in an experimental example.

### Description of Embodiments

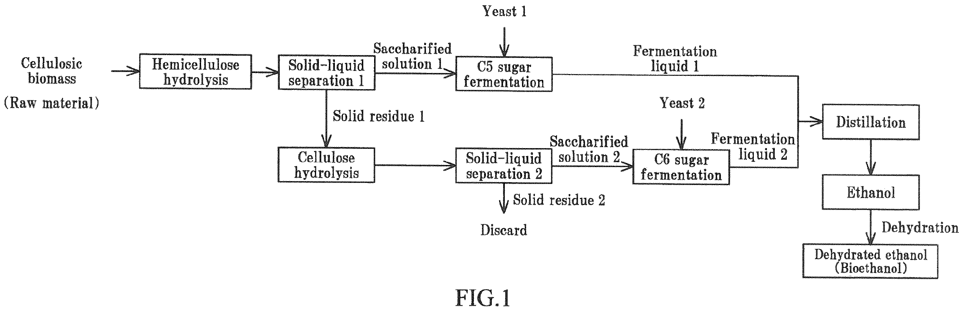

An embodiment of the present invention will be described with reference to the drawings. FIG. 1 shows a basic flow of a process of producing a saccharified solution from cellulosic biomass by an enzymatic technique.

### <Pre-treatment step>

First, cellulosic biomass (hereinafter referred to as "biomass") such as sugarcane bagasse is coarsely pulverized with a crusher or grinder. The cellulosic biomass is preferably pulverized into pieces with an average diameter of 10 to 100 mm. The coarsely pulverized biomass may be subjected to digesting treatment using an aqueous solution of a metal hydroxide such as sodium hydroxide. If the digesting treatment is performed, part of lignin contained in the bagasse is removed, and the reactivity of cellulose and hemicellulose with enzymes is increased. In this case, the biomass subjected to the digesting treatment is neutralized with an acid.

Instead of the digesting treatment using an aqueous solution of a metal hydroxide, steam explosion treatment using an explosion treatment device may be performed at 200 to 240°C and 1.5 to 4 MPa for 1 to 15 min (preferably at 225 to 230°C and 2.5 to 3 MPa for 1 to 5 min) to accomplish saccharification of a hemicellulose component in the biomass and partial removal of lignin from the biomass. The biomass treated by the explosion treatment device (hereinafter referred to as "explosion-treated product") contains a C5 saccharified solution derived from hemicellulose, dissolved lignin, and a solid residue. When the C5 saccharified solution is to be subjected to hemicellulosic sugar fermentation separately from a C6 saccharified solution, the explosion-treated product is subjected to solid-liquid separation with means such as a filter press and separated into an explosion-treated solution and the solid residue. The solid residue may be washed with water as needed to collect sugars contained in the solid residue. When C5 sugar fermentation and C6 sugar fermentation are simultaneously performed, the solid-liquid separation may or may not be conducted.

When the explosion treatment is performed, the solid residue obtained through the explosion treatment may be washed with water to remove sugars and dissolved lignin and may then be immersed in an aqueous ethanol solution having an ethanol concentration of 30% or more, preferably 50% or more, at normal temperature for a time of 0.5 to 48 hours, preferably 1 to 24 hours, to dissolve and remove lignin covering cellulose. After the ethanol immersion, the solid residue is subjected to ethanol removal treatment with mechanical means or by heating or heating under reduced pressure. When the aqueous ethanol solution used has a high concentration, the solid residue may be washed with water before the ethanol removal treatment.

### <Hemicellulose hydrolysis (saccharification)>

Hemicellulose contained in the cellulosic biomass may be hydrolyzed with a hemicellulose-hydrolyzing enzyme such as hemicellulase, or may alternatively be hydrolyzed using high-pressure hot water (hot water treatment). Hydrolysis using high-pressure hot water at 140 to 180°C can hydrolyze hemicellulose into sugars (mainly C5 monosaccharides). When the biomass has a high hemicellulose content, treatment at high temperature causes excessive hydrolysis of the C5 monosaccharides into organic acids etc. Thus, the hydrolysis treatment is preferably carried out under relatively mild conditions.

It is considered preferable, when hemicellulose is hydrolyzed using high-pressure hot water, to add an acid such as phosphoric acid or hydrochloric acid as a catalyst. When hemicellulose is hydrolyzed with a hemicellulose-hydrolyzing enzyme, a commercially-available enzyme may be used, or a microorganism that produces hemicellulase may be used.

### <Solid-liquid separation 1>

The hydrolysis of hemicellulose is followed by solid-liquid separation 1 to separate a solid residue 1 and a saccharified solution 1 (C5 saccharified solution) from each other.

### <C5 sugar fermentation>

A yeast is added to the saccharified solution 1, in which hemicellulosic sugar fermentation proceeds to give an alcohol (fermentation liquid 1).

### <Cellulose hydrolysis (saccharification)>

To the solid residue 1 are added water and a cellulose-hydrolyzing enzyme such as cellulase, and thus cellulose contained in the solid residue 1 is hydrolyzed. When cellulose is hydrolyzed with a cellulose-hydrolyzing enzyme, a commercially-available enzyme may be used, or a microorganism that produces cellulase may be used.

### <Solid-liquid separation 2>

The hydrolysis of cellulose is followed by solid-liquid separation 2 to separate a solid residue 2 and a saccharified solution 2 (C6 saccharified solution) from each other. The solid residue is washed with water as needed to collect C6 sugars and then discarded.

### <C6 sugar fermentation>

A yeast is added to the saccharified solution 2, in which cellulosic sugar fermentation proceeds to give an alcohol (fermentation liquid 2).

While in FIG. 1 the C5 sugar fermentation and C6 sugar fermentation are carried out independently of each other, the saccharified solution 1 and saccharified solution 2 may be mixed, and the C5 sugar fermentation and C6 sugar fermentation may be allowed to proceed simultaneously in the mixture of the saccharified solutions.

### <Distillation and dehydration>

The alcoholic fermentation liquids (fermentation liquid 1 and fermentation liquid 2) resulting from alcoholic fermentation of the C5 and C6 saccharified solutions are delivered to a distillation device, in which the fermentation liquids are distilled to give ethanol. The resulting ethanol is dehydrated with a known ethanol dehydration agent or ethanol dehydration device, and the dehydrated ethanol is shipped as an end product (bioethanol).

In FIG. 1, the solid-liquid separation 1 or solid-liquid separation 2 may be skipped, and the hemicellulosic sugar fermentation and cellulosic sugar fermentation may be performed with the solid residue remaining in the saccharified solution.

### (Step A)

Hereinafter, the steps of the saccharified solution production method of the present invention will be described. First, cellulosic biomass is coarsely pulverized, and then hemicellulose contained in the pulverized cellulosic biomass is hydrolyzed by hot water treatment or with a hemicellulose-hydrolyzing enzyme. The step A is followed by solid-liquid separation using a solid-liquid separation device to separate a C5 saccharified solution and a solid residue from each other.

### (Step B)

Next, the solid residue, from which hemicellulose has been removed, is mixed with an aqueous solution containing a cellulose-hydrolyzing enzyme in a first reaction vessel. The first reaction vessel includes no baffle plate disposed in its interior. The bottom surface of the first reaction vessel may be flat, conical, or dished, and the material, dimensions, and other properties of the vessel can be selected as appropriate depending on the amount of the cellulosic biomass to be processed.

In the interior of the first reaction vessel, a plurality of impellers of a stirring device are disposed at least in a region where the impellers can contact the contents of the vessel. The ratio of the rotation diameter of the impellers to the inner diameter of the first reaction vessel is preferably large. Each impeller of the stirring device is preferably a helical ribbon impeller, a double helical ribbon impeller, a Maxblend impeller, an inclined paddle impeller, or an anchor impeller. The plurality of impellers of the stirring device may be of the same or different types. The impellers are preferably rotated at an impeller tip speed of 0.5 to 5.0 m/s.

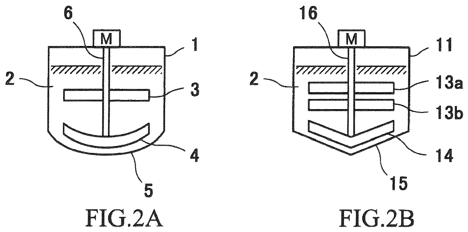

FIGS. 2A and 2B are schematic configuration diagrams showing examples of the first reaction vessel suitable for carrying out the step B. A first reaction vessel 1 shown in FIG. 2A includes impellers 3 and 4 and has a dished bottom surface 5. The bottom surface 5 may be flat. A rotary shaft 6 is rotated by a motor M mounted on the top surface of the reaction vessel 1.

A first reaction vessel 11 shown in FIG. 2B includes impellers 13a, 13b, and 14. The impellers 13a and 13b are of the same type. The first reaction vessel 11 has a conical bottom surface 15. A rotary shaft 16 is rotated by a motor M mounted on the top surface of the reaction vessel 11.

Into the first reaction vessel are introduced an aqueous solution containing a cellulose-hydrolyzing enzyme and a solid residue from which hemicellulose has been removed. A necessary amount of water may be first introduced into the vessel, and then the cellulose-hydrolyzing enzyme may be added and mixed with the water to prepare the aqueous solution of the cellulose-hydrolyzing enzyme. The solids concentration in the reaction vessel is adjusted so that the final concentration is 15 to 30% by mass. Afterwards, the contents of the vessel are stirred by the impellers of the stirring device for 5 minutes to 24 hours. Through this process, the solid residue (cellulosic biomass) from which hemicellulose has been removed is solubilized.

The step B is preferably continued until the contents of the first reaction vessel attain a flowability high enough to allow delivery by a centrifugal pump (typically, until the viscosity of the contents decreases to 300 cp or less). After this step, the contents of the first reaction vessel are transferred by a pump or pneumatically to a second reaction vessel connected to the first reaction vessel via a pipe, the second reaction vessel including a baffle plate disposed in its interior. The second reaction vessel may have the same structure as the first reaction vessel, except that the second reaction vessel includes a baffle plate and at least one impeller which are disposed in its interior.

The step B is preferably preceded by a step A1 of subjecting the solid residue, from which hemicellulose has been removed, to the digesting treatment or steam explosion treatment described above and thereby removing lignin from the solid residue. The step A1 may be performed before the step A or between the step A and step B.

### (Step C)

The contents of the second reaction vessel (a mixture of the solid residue from which hemicellulose has been removed and the aqueous solution containing the cellulose-hydrolyzing enzyme) are stirred by the stirring device to allow a hydrolysis reaction to proceed until most of contained cellulose is converted to glucose (in other words, until the cellulose hydrolysis reaction of the contents of the reaction vessel is completed). In the step C, the stirring is performed for a time of 5 minutes to 120 hours while the contents of the reaction vessel are adjusted to a temperature of 20 to 90°C. In the step C, cellulose contained in the solid residue is hydrolyzed, and the contents of the reaction vessel are formed into a slurry.

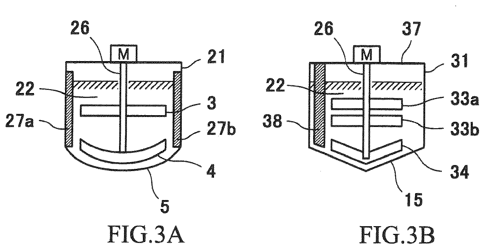

FIGS. 3A and 3B are schematic configuration diagrams showing examples of the second reaction vessel. A second reaction vessel 21 shown in FIG. 3A has an inner wall on which baffle plates 27a and 27b are mounted. The baffle plates may be disposed at any other locations which are inside the second reaction vessel 21 and at which the baffle plates can contact the vessel contents during stirring. While it is preferable to mount a plurality of baffle plates, only a single baffle plate may be mounted if the baffle plate can enhance the efficiency of stirring of the contents. The dimensions of the baffle plates may be any dimensions commonly employed for use in reaction vessels. Such dimensions are specified, for example, in "Kagaku Kogaku Binran (Handbook of Chemical Engineering)" edited by The Society of Chemical Engineers, Japan.

The baffle plate disposed in the second reaction vessel may have a shape similar to that of baffle plates commonly used in reaction vessels. For example, the width of the baffle plate is about 1/12 to 1/10 of the inner diameter of the reaction vessel, and the thickness of the baffle plate is 6 mm or more in view of mechanical strength. Any other limitations are not imposed on the dimensions, material, and other properties of the baffle plate.

A second reaction vessel 31 shown in FIG. 3B includes a baffle plate 38 suspended from a top surface 37. The baffle plate 38 is not in contact with the inner wall of the second reaction vessel 31. While in FIG. 3B the baffle plate 38 is disposed only on the left side of the figure, another baffle plate may be disposed on the right side of the figure in such a manner that the two baffle plates are symmetrically located. Three or more baffle plates may be disposed.

In the step C, the vessel contents have a viscosity of 300 cp or less, which is lower than that in the step B. If a baffle plate is disposed in the first reaction vessel for the step B, the solids will be readily deposited on the baffle plate, and the flow of the vessel contents will thus be hindered by the baffle plate, so that the efficiency of stirring of the vessel contents will be reduced. In contrast, for the second reaction vessel for the step C, disposing the baffle plate in the interior of the vessel provides the following advantage: when the vessel contents are stirred to cause a turbulent flow with a low Reynolds number, not only a swirling flow in the horizontal direction but a convection flow in the vertical direction are generated in the vessel contents, and the efficiency of stirring of the vessel contents can be enhanced. Consequently, reduction in the rate of the enzymatic hydrolysis reaction of cellulose can be prevented.

### (Step D)

After the step C, the slurry is withdrawn from the second reaction vessel. The withdrawn slurry is subjected to solid-liquid separation as needed to separate the slurry into a solid residue and a C6 saccharified solution. The C6 saccharified solution alone or mixed with the C5 saccharified solution is subjected to ethanol fermentation or lactic acid fermentation as a raw material for bioethanol or bio-lactic acid.

### [Experimental example]

167 g of pulverized bagasse having a water content of 10% and 333 g of water were placed in a pressure vessel, and the mixture was adjusted to a pH of 3 with phosphoric acid. The pH adjustment was followed by hot water treatment at 1.1 MPaG and 180°C for 10 minutes, and the hot water-treated residue was dehydrated using filtration paper and an aspirator and thus adjusted to a water content of 30%. To the dehydrated, hot water-treated residue with a wet weight of 25 g were added 20 g of a 0.05 M citric acid buffer solution, 1 g of a commercially-available aqueous cellulase solution (final filter paper unit: 15 FPU/mg-biomass), and 4 g of water, and the mixture with a total weight of 50 g was placed in a screw vial with an overall volume of 100 mL.

Afterwards, the screw vial was placed in a shaker thermostat adjusted to 50°C, and the reaction was allowed to proceed for 72 h. The contents of the screw vial correspond to the contents of the second reaction vessel in the step C. Two tests were conducted for comparison by varying the shaking rate; in one test, the shaking rate was set to 150 rpm, so that the solids as visually observed were dispersed uniformly (this test corresponds to the case where a baffle plate is disposed), while in the other test the shaking rate was set to 75 rpm, so that the solids settled on the bottom of the vial (this test corresponds to the case where no baffle plate is disposed).

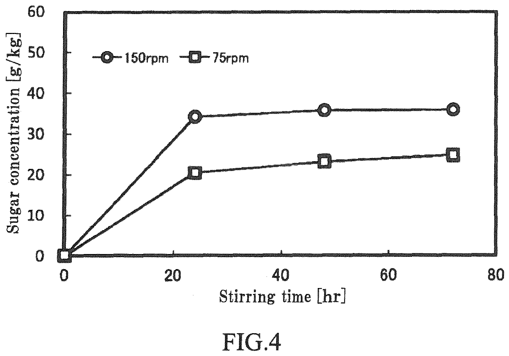

FIG. 4 is a graph plotted for the relationship between the stirring time and the sugar concentration of the saccharified solution (the contents of the screw vial). It is seen that the sugar concentration at a stirring time of 72 h was about 40% higher for 150 rpm than for 75 rpm.

It has thus been demonstrated that when, in the step C, a baffle plate is disposed in the reaction vessel to enhance the efficiency of stirring of the reaction vessel contents, the amount of sugar production can be markedly increased without the use of an expensive device and without an increase in running cost.

### Industrial Applicability

The saccharified solution production method of the present invention is beneficial as a saccharified solution production method for use in the field of biochemical substances such as bioethanol and bio-lactic acid.

### Reference Signs List

- 1, 11:: first reaction vessel
- 2:: contents of first reaction vessel
- 3:: impeller (upper impeller)
- 4:: impeller (lower impeller)
- 5:: dished bottom surface
- 6:: rotary shaft of first stirring device
- 13a:: impeller (upper impeller) of first stirring device
- 13b:: impeller (central impeller) of first stirring device
- 14:: impeller (lower impeller) of first stirring device
- 15:: conical bottom surface
- 21, 31:: second reaction vessel
- 22:: contents of second reaction vessel
- 26:: rotary shaft of second stirring device
- 27a, 27b, 38:: baffle plate
- 33a:: impeller (upper impeller) of second stirring device
- 33b:: impeller (central impeller) of second stirring device
- 34:: impeller (lower impeller) of second stirring device
- 37:: top surface of second reaction vessel
- M:: motor

## Claims

1. A saccharified solution production method for obtaining a saccharified solution by enzymatic hydrolysis of cellulosic biomass, the method comprising the steps of:
A) hydrolyzing hemicellulose contained in the cellulosic biomass by hot water treatment or with a hemicellulose-hydrolyzing enzyme;
B) mixing a solid residue, from which hemicellulose has been removed, with an aqueous solution containing a cellulose-hydrolyzing enzyme in a first reaction vessel, adjusting the resulting mixture to a solids concentration of 15 to 30% by mass, and then stirring the mixture with a first stirring device disposed in an interior of the first reaction vessel to solubilize the solid residue from which hemicellulose has been removed;
C) transferring the contents of the first reaction vessel to a second reaction vessel after the step B and stirring the contents of the second reaction vessel with a second stirring device disposed in an interior of the second reaction vessel to allow a hydrolysis reaction to proceed; and
D) withdrawing a slurry obtained as a result of the hydrolysis reaction from the second reaction vessel, wherein
the first reaction vessel includes no baffle plate disposed in the interior thereof, and
the second reaction vessel includes a baffle plate disposed in the interior thereof.

2. The saccharified solution production method according to claim 1, wherein
the first stirring device includes a plurality of impellers disposed at least in the interior of the first reaction vessel, and
the second stirring device includes a single impeller disposed at least in the interior of the second reaction vessel.

3. The saccharified solution production method according to claim 1 or 2, wherein
each of the first stirring device and the second stirring device includes an impeller selected from the group consisting of a helical ribbon impeller, a double helical ribbon impeller, a Maxblend impeller, an inclined paddle impeller, and an anchor impeller, and
the impeller of the first stirring device is rotated at an impeller tip speed of 0.5 to 5.0 m/s.

4. The saccharified solution production method according to any one of claims 1 to 3, further comprising a step Al of removing lignin contained in the cellulosic biomass before the step B.
